# FASCICULE DE BREVET EUROPEEN

(11) **EP 0 662 315 B1**
(45) Date de publication et mention de la délivrance du brevet: **27.11.1996**
(21) Numéro de dépôt: 94402693.9
(22) Date de dépôt: 24.11.1994
(51) Int. Cl.: A61K 7/06, A61K 7/50

(54) **Compositions cosmétiques détergentes à usage capillaire contenant des alpha-hydroxyacides**
Alpha-Hydroxysäuren enthaltendes kosmetisches Shampoos
Cosmetic shampoos containing alpha-hydroxyacids

(30) Priorité: 11.01.1994 FR 9400219
(43) Date de publication de la demande: 12.07.1995
(73) Titulaire: L'OREAL, 75008 Paris (FR)
(72) Inventeur: Beauquey, Bernard, F-92110 Clichy (FR); Decoster, Sandrine, F-93800 Epinay sur Seine (FR); Cauwet, Danièle, F-75011 Paris (FR); Dubief, Claude, F-78150 Le Chesnay (FR)
(74) Mandataire: Andral, Christophe André Louis

(56) Documents cités:
- EP-A- 0 403 304
- EP-A- 0 508 324
- EP-A- 0 531 943

## Description

La présente invention concerne de nouvelles compositions cosmétiques à propriétés améliorées destinées au nettoyage ou au soin des cheveux et/ou du cuir chevelu, à base de tensio-actifs anioniques, de tensio-actifs non-ioniques, d'agents conditionneurs et d'acides carboxyliques α-hydroxylés ou leurs dérivés, ainsi que leur utilisation dans cette application cosmétique.

Pour le nettoyage et/ou le soin des cheveux, l'utilisation de compositions capillaires détergentes (ou shampooings) contenant à la fois des agents tensio-actifs anioniques et des agents tensio-actifs non-ioniques (mélanges), est courante.

On sait en effet que ces compositions possèdent un excellent pouvoir lavant, mais les propriétés cosmétiques intrinsèques qui leur sont attachées restent toutefois relativement faibles.

Aussi, pour améliorer les propriétés cosmétiques des compositions détergentes ci-dessus, et plus particulièrement de celles qui sont appelées à être appliquées sur des cheveux sensibilisés (i.e. des cheveux qui se trouvent abimés ou fragilisés notamment sous l'action chimique des agents atmosphériques et/ou de traitements capillaires tels que permanentes, teintures ou décolorations), il est connu d'introduire dans ces dernières des agents cosmétiques complémentaires dits agents conditionneurs (polymères notamment), comme par exemple des polymères cationiques, des polymères amphotères ou bien encore des silicones et/ou des dérivés de silicones, qui apportent alors aux cheveux traités une facilité de démélage et de coiffage, ainsi qu'une douceur, nettement accrues.

Cependant, pour de telles compositions détergentes à base de tensio-actifs anioniques et non-ioniques et d'agents conditionneurs, on observe, de façon inexplicable, que les avantages cosmétiques mentionnés ci-avant s'accompagnent malheureusement également, sur cheveux séchés, de certains effets cosmétiques jugés indésirables, à savoir un alourdissement de la coiffure (manque de légéreté du cheveu), un manque de lissage (cheveu non homogène de la racine à la pointe) et une insuffisance concernant la brillance. Par ailleurs, on constate que ces inconvénients se trouvent fortement accentués en cas d'application de la composition détergente sur des cheveux sensibilisés ; en outre, si ces cheveux ont été sensibilisés par des traitements de colorations, en particulier de colorations d'oxydation, l'utilisation répétée de la composition détergente induit à la longue une diminution néfaste de la tenue mécanique des cheveux.

En résumé, il s'avère que les compositions détergentes actuelles contenant des tensio-actifs anioniques, des tensio-actifs non-ioniques et des agents conditionneurs, ne donnent pas complètement satisfaction.

Or, à la suite d'importantes recherches menées sur la question, il a maintenant été trouvé par la Demanderesse qu'en introduisant, en une quantité suffisante, des acides carboxyliques α-hydroxylés dans les compositions capillaires détergentes de l'art antérieur à base de tensio-actifs anioniques, de tensio-actifs non-ioniques et d'agents conditionneurs, il est possible de limiter, voire supprimer, les problèmes généralement liés à l'emploi de telles compositions, à savoir en particulier l'alourdissement, le manque de lissage et de brillance, et l'amoindrissement de la tenue mécanique, des cheveux, tout en conservant le pouvoir lavant et les autres propriétés cosmétiques avantageuses (douceur, démélage,coiffage) qui sont attachés à ces dernières.

Cette découverte est à la base la présente invention.

Ainsi, selon la présente invention, il est maintenant proposé de nouvelles compositions capillaires détergentes, du type comprenant, dans un milieu cosmétiquement acceptable, au moins un agent tensio-actif anionique, au moins un agent tensio-actif non-ionique et au moins un agent conditionneur, et qui sont caractérisées par le fait qu'elles comprennent en outre, à raison d'au moins 2% en poids par rapport à l'ensemble de la composition, au moins un acide carboxylique présentant un radical hydroxy en position α ou les dérivés de cet acide.

L'invention a également pour objet l'utilisation en cosmétique des compositions ci-dessus pour le nettoyage ou le soin des cheveux et/ou du cuir chevelu.

D'autres caractéristiques, aspects et avantages de l'invention apparaitront encore plus clairement à la lecture de la description qui va suivre ainsi que des exemples concrets, mais nullement limitatifs, destinés à l'illustrer.

La nature du tensio-actif anionique rentrant dans les compositions détergentes selon l'invention n'est pas critique. Ainsi, à titre d'exemple de tensio-actifs anioniques utilisables, seuls ou mélanges, dans le cadre de la présente invention, on peut citer notamment (liste non limitative) les sels (en particulier sels alcalins, notamment de sodium, sels d'ammonium, sels d'amines, sels d'aminoalcools ou sels de magnésium) des composés suivants : les alkylsulfates, les alkyléthersulfates, alkylamidoéthersulfates, alkylarylpolyéthersulfates, monoglycérides sulfates ; les alkylsulfonates, alkylsulfates, alkylphosphates, alkylamidesulfonates, alkylarylsulfonates, α-oléfine-sulfonates, paraffine-sulfonates ; les alkylsulfosuccinates, les alkyléthersulfosuccinates, les alkylamidesulfosuccinates; les alkylsulfosuccinamates ; les alkylsulfoacétates ; les alkylétherphosphates; les acylsarcosinates et les N-acyltaurates, le radical alkyle ou acyle de tous ces différents composés comportant de préférence de 12 à 20 atomes de carbone. Parmi les tensioactifs anioniques encore utilisables, on peut également citer les sels d'acides gras tels que les sels des acides oléique, ricinoléique, palmitique, stéarique, les acides d'huile de coprah ou d'huile de coprah hydrogénée ; les acyl-lactylates dont le radical acyle comporte 8 à 20 atomes de carbone. On peut également utiliser des tensioactifs faiblement anioniques, comme les acides d'alkyl D-galactoside uroniques et leurs sels ainsi que les acides éthers carboxyliques polyoxyalkylénés, en particulier ceux comportant de 2 à 24 groupements oxyde d'éthyléne, et leurs mélanges.

Les agents tensioactifs non-ioniques sont, eux aussi, des composés bien connus en soi (voir notamment à cet égard "Handbook of Surfactants" par M.R. PORTER, éditions Blackie & Son (Glasgow and London), 1991, pp 116-178) et leur nature ne revet pas, dans le cadre de la présente invention, de caractère critique. Ainsi, ils peuvent être notamment choisis parmi les alcools, les alphadiols, les alkylphénols ou les acides gras polyéthoxylés, polypropoxylés ou polyglycérolés, ayant une chaîne grasse comportant par exemple 8 à 18 atomes de carbone, le nombre de groupements oxyde d'éthylène ou oxyde de propylène pouvant aller notamment de 2 à 50 et le nombre de groupements glycérol pouvant aller notamment de 2 à 30. On peut également citer les copolymères d'oxyde d'éthylène et de propylène, les condensats d'oxyde d'éthylène et de propylène sur des alcools gras ; les amides gras polyéthoxylés ayant de préférence de 2 à 30 moles d'oxyde d'éthyléne, les amides gras polyglycérolés comportant en moyenne 1 à 5 groupements glycérol et en particulier 1,5 à 4 ; les amines grasses polyéthoxylées ayant de préférence 2 à 30 moles d'oxyde d'éthylène ; les esters d'acides gras du sorbitan oxyéthylénés ayant de 2 à 30 moles d'oxyde d'éthylène ; les esters d'acides gras du sucrose, les esters d'acides gras du polyéthylèneglycol, les alkylpolyglycosides, les dérivés de N-alkyl glucamine, les oxydes d'amines tels que les oxydes d'alkyl (C₁₀ - C₁₄) amines ou les oxydes de N-acylaminopropylmorpholine. On notera que les alkylpolyglycosides constituent des tensio-actifs non-ioniques rentrant particulièrement bien dans le cadre de la présente invention.

Les agents conditionneurs utilisables selon la présente invention peuvent être choisis parmi tous ceux déja connus en soi comme améliorant les propriétés cosmétiques des cheveux traités par des compositions détergentes, à savoir notamment des polymères cationiques, tels que ceux par exemple décrits dans la demande de brevet EP-A- 0 337 354 et les demandes de brevets français FR-A- 2 270 846, 2 383 660, 2 598 611, 2 470 596 et 2 519 863 ; des polymères amphotères tels que ceux par exemple décrits dans la demande de brevet EP-A- 0 269 243, le brevet US 3 836 537, la demande de brevet EP-A- 0 269 243 et les deux dernières demandes de brevets français précitées ; des silicones et leurs dérivés, telles que celles par exemple décrites dans les demandes de brevets EP-A- 0 398 177 et EP-A- 0 492 657 ; et, mais dans une bien moindre mesure, certains polymères anioniques.

Les compositions détergentes conformes à l'invention peuvent bien entendu contenir un ou plusieurs agents tensio-actifs anioniques, un ou plusieurs agents tensio-actifs non-ioniques et un ou plusieurs agents conditionneurs.

Selon une caractéristique essentielle des compositions capillaires détergentes selon l'invention, ces dernières contiennent au moins un acide carboxylique hydroxylé en position α (composé encore appelé α (alpha) hydroxyacide) ou ses dérivés.

Par dérivés d'acide, on entend ses sels associés (sels avec une base organique ou un alcalin notamment) ou bien encore éventuellement son lactide correspondant (forme obtenu par autoestérification des molécules).

Cet α hydroxyacide peut bien entendu consister en un acide mono ou poly carboxylique comportant une ou plusieurs fonctions hydroxy, l'une au moins de ces fonctions hydroxy devant occuper une position α sur ledit acide (carbone adjacent à une fonction carboxylique). Cet acide peut se présenter dans la composition détergente finale sous forme d'acide libre et/ou sous la forme de l'un de ses sels associés (sels avec une base organique ou un alcalin notamment), en particulier selon le pH final imposé à la composition, ou bien encore éventuellement sous la forme du lactide correspondant (forme obtenu par autoestérification des molécules). Les compositions détergentes conformes à l'invention peuvent bien entendu contenir un plusieurs α hydroxyacides.

A titre d'exemples de tels composés, on peut citer, entre autres, les acides citrique, lactique, méthyllactique, phényllactique, malique, mandélique, glycolique, tartronique, tartrique, gluconique, benzylique et l'acide 2-hydroxycaprylique. D'autres composés de type α hydroxyacides convenant à la présente invention sont ceux cités dans la demande de brevet EP-A- 0 413 528.

De préférence, on retiendra les acides qui sont cosmétiquement compatibles et acceptables avec les cheveux, la peau et/ou le cuir chevelu.

Selon un mode particulièrement préféré de réalisation de la présente invention, l'α hydroxyacide mis en oeuvre est choisi parmi l'acide citrique, l'acide tartrique et l'acide lactique.

Selon une autre caractéristique importante des compositions détergentes selon l'invention, le ou les α hydroxyacides sont présents dans ces dernières à raison d'au moins 2 % en poids, de préférence au moins 3 % en poids par rapport à l'ensemble de la composition. Des teneurs en α hydroxyacide(s) comprises entre 2 et 10 % en poids, et plus particulièrement encore entre 4 et 5 % en poids, conviennent généralement très bien. On notera que ces concentrations sont nettement supérieures à celles qui peuvent parfois se rencontrer dans des shampooings de l'art antérieur lorsque certains acides ont été employés uniquement à des fins d'ajustement de pH.

A titre indicatif, les formulations détergentes conformes à l'invention présentent généralement les compositions suivantes :
(i) tensio-actif(s) anionique(s) : de 5 à 50 % en poids, de préférence de 5 à 20 % en poids, par rapport au poids total de la formulation détergente ;
(ii) tensio-actif(s) non-ionique(s) : de 5 à 30 % en poids, et de préférence de 5 à 20 % en poids, par rapport au poids total de la formulation détergente ;
(iii) agent(s) conditionneur(s) : de 0,01 à 10 % par rapport au poids total de la formulation détergente ;
(iv) α hydroxyacide(s) : comme indiqué ci-avant.

Le véhicule, ou support, des compositions détergentes selon l'invention est de préférence de l'eau ou une solution hydroalcoolique d'un alcool inférieur tel que éthanol, isopropanol ou butanol.

Les compositions détergentes selon l'invention présentent un pH final généralement compris entre 2 et 8. De préférence, ce pH est compris entre 3 et 7. L'ajustement du pH à la valeur désirée peut se faire classiquement par ajout d'une base (organique ou minérale) dans la composition, par exemple de l'ammoniaque ou une (poly)amine primaire, secondaire ou tertiaire comme la monoéthanolamine, la diéthanolamine, la triéthanolamine, l'isopropanolamine ou la propanediamine-1,3. De préférence, on met en oeuvre l'ammoniaque. La neutralisation de l'α hydroxyacide par une base présente pour avantage complémentaire de favoriser la formation d'un tampon qui, en évitant les fluctuations de pH, permet d'améliorer la stabilité du shampooing ; par ailleurs, cette neutralisation engendre généralement un épaississement spontané de ce dernier, ce qui est souvent recherché dans ce type d'application.

Les compositions détergentes selon l'invention peuvent bien entendu contenir en outre tous les adjuvants usuels rencontrés dans le domaine des shampooings, comme par exemple des parfums, des agents conservateurs, des sequestrants, des épaississants, des adoucissants, des modificateurs de mousse, des colorants, des agents nacrants, des agents hydratants, des agents anti-pélliculaires ou anti-séborrhéiques, des vitamines, des filtres solaires et autres.

Ces compositions peuvent se présenter sous la forme de liquides plus ou moins épaissis, de crêmes ou de gel et elles conviennent principalement au lavage, au soin et/ou la beauté des cheveux.

Des exemples concrets illustrant l'invention vont maintenant être donnés.

### EXEMPLE

On a préparé et testé trois shampoings conformes à l'invention (A, B et C) qui présentaient les compositions suivantes :

### Shampooing A :

| | |
|---|---|
| - Lauryl éther sulfate de sodium et magnésium à 4 moles d'oxyde d'éthylène, vendu sous le nom de TEXAPON ASV par HENKEL (tensio-actif anionique) | 6,5 g |
| - Caprilyl-capryl éther de glucoside, vendu sous le nom de ORAMIX CG 110 par SEPPIC (tensio-actif non-ionique) | 15 g |
| - Copolymère vinylpyrrolidone/méthacrylate de diméthylaminoéthyl quaternisé par du sulfate de diéthyl, vendu sous le nom de GAFQUAT 755 par la Société ISP (agent conditionneur) | 0,2 g |
| - EDTA | 0,25 g |
| - Conservateurs | 0,31 g |
| - Acide citrique | 5 g |
| - Eau qsp | 100 g |

Le pH de ce shampooing a été ajusté à pH 5 par ajout d'ammoniaque.

### Shampoing B :

| | |
|---|---|
| - Lauryl éther sulfate de sodium à 2,2 moles d'oxyde d'éthylène (tensio-actif anionique) | 5,6 g |
| - Dodécanediol polyglycérolé à 3,5 moles de glycérol, préparé selon FR-A-2 091 516 (tensio-actif non-ionique) | 10 g |
| - Hydroxyéthyl cellulose réticulée et quaternisée, vendue sous le nom de JR 400 par la Société UNION CARBIDE (agent conditionneur) | 0,2 g |
| - Suif oxyéthyléné (60 moles d'oxyde d'éthylène) éther de myristyl glycol, vendu sous le nom de ELFACOS GT 282 S par AKZO (agent épaississant) | 1,5 g |
| - EDTA | 0,25 g |
| - Conservateurs | 0,31 g |
| - Acide citrique | 5 g |
| - Eau qsp | 100g |

Le pH de ce shampooing a été ajusté à pH 5 par ajout d'ammoniaque.

### Shampooing C :

| | |
|---|---|
| - Lauryl éther sulfate de sodium à 2,2 moles d'oxyde d'éthylène (tensio-actif anionique) | 4,2 g |
| - Alcool laurique oxyéthyléné à 12 moles d'oxyde d'éthylène, vendu sous le nom de LAUROPAL 12 par WITCO (tensio-actif non-ionique) | 7 g |
| - Copolymère vinylpyrrolidone/méthacrylate de diméthylaminoéthyl quaternisé par du sulfate de diéthyl, vendu sous le nom de GAFQUAT 755 par la Société ISP (agent conditionneur) | 0,2 g |
| - Diéthanolamide d'acide de coprah (épaississant) | 3 g |
| - EDTA | 0,25 g |
| - Conservateurs | 0,31 g |
| - Acide citrique | 5 g |
| - Eau qsp | 100g |

Le pH de ce shampooing a été ajusté à pH 5 par ajout d'ammoniaque.

Les performances cosmétiques des shampooings ci-dessus ont été évaluées par un panel d'experts à l'aide de tests d'analyses sensorielles conduits in vivo sur des cheveux humains sensibilisés (ici, des cheveux permanentés). Le mode opératoire était le suivant : les shampooings sont appliqués sur cheveux humides, puis émulsionnés et enfin rincés à l'eau après quelques minutes de pause ; après séchage de la chevelure, les cheveux sont examinés par les experts.

A titre comparatif, on a également testé, selon le même protocole, trois compositions A', B' et C' identiques respectivement aux compositions A, B et C ci-dessus, à cette seule différence près que toutes trois étaient cette fois exemptes d' α hydroxyacides et qu'elles ont été amenées au même pH que celui des compositions de l'invention correspondantes par ajout d'acide chlorhydrique.

Le panel d'experts a jugé et conclu que les compositions A, B et C conformes à l'invention présentaient, par rapport aux compositions comparatives correspondantes, les effets bénéfiques représentatifs suivants : apport de légéreté, de souplesse, de lissage et de brillance sur cheveux secs.

Par ailleurs, des tests de résistance mécaniques conduits sur des cheveux qui avaient été traités de façon répétitive plusieurs dizaines de fois avec les shampooings comparatifs d'une part et les shampooings conformes à l'invention d'autre part, ont montré que, dans ce dernier cas, il n'y avait aucune chute notable de la tenue mécanique des cheveux.

## Revendications

1. Compositions capillaires détergentes, du type comprenant, dans un milieu cosmétiquement acceptable, au moins un agent tensio-actif anionique, au moins un agent tensio-actif non-ionique et au moins un agent conditionneur, caractérisées par le fait qu'elles comprennent en outre, à raison d'au moins 2% en poids par rapport à l'ensemble de la composition, au moins un acide carboxylique présentant un radical hydroxy en position α (alpha hydroxyacide) ou ses dérivés.

2. Compositions selon la revendication 1, caractérisées par le fait que ladite teneur est d'au moins de 3 % en poids.

3. Compositions selon l'une quelconque des revendications précédentes, caractérisées par le fait que ladite teneur est inférieure à 10 % en poids.

4. Compositions selon l'une quelconque des revendications précédentes, caractérisées par le fait que le ou les tensio-actifs anioniques sont présents à raison de 5 à 50 % en poids, de préférence de 5 à 20 % en poids, par rapport au poids total de la composition.

5. Compositions selon l'une quelconque des revendications précédentes, caractérisées par le fait que le ou les tensio-actifs non-ioniques sont présents à raison de 5 à 30 % en poids, de préférence de 5 à 20 % en poids, par rapport au poids total de la composition.

6. Compositions selon l'une quelconque des revendications précédentes, caractérisées par le fait que le ou les agents conditionneurs sont présents à raison de 0,01 à 10 % en poids par rapport au poids total de la composition.

7. Compositions selon l'une quelconque des revendications précédentes, caractérisées par le fait qu'il s'agit de compositions aqueuses ou hydroalcooliques.

8. Compositions selon l'une quelconque des revendications précédentes, caractérisées par le fait qu'elles présentent un pH compris entre 2 et 8.

9. Compositions selon la revendication 8, caractérisées par le fait que ledit pH est compris entre 3 et 7.

10. Compositions selon l'une quelconque des revendications précédentes, caractérisées par le fait que l'alpha hydroxyacide ou ses dérivés est présent sous forme d'acide libre et/ou sous la forme de l'un de ses sels associés et/ou sous la forme du lactide correspondant.

11. Compositions selon l'une quelconque des revendications précédentes, caractérisées par le fait que l'alpha hydroxyacide est choisi parmi l'acide citrique, l'acide tartrique et l'acide lactique.

12. Compositions selon l'une quelconque des revendications précédentes, caractérisées par le fait que l'agent conditionneur est choisi parmi les polymères cationiques, les polymères amphotères, les silicones et les dérivés de silicone.

13. Utilisation d'une composition telle que définie à l'une quelconque des revendications précédentes pour le nettoyage et/ou le soin des cheveux et/ou du cuir chevelu.

14. Utilisation selon la revendication 13, caractérisée par le fait que lesdits cheveux sont des cheveux sensibilisés.

## Claims

1. Detergent hair compositions of the type comprising, in a cosmetically acceptable medium, at least one anionic surface-active agent, at least one nonionic surface-active agent and at least one conditioning agent, characterized in that they additionally comprise, in an amount of at least 2% by weight relative to the whole of the composition, at least one carboxylic acid having a hydroxyl radical in the α position (alpha-hydroxy acid) or its derivatives.

2. Compositions according to Claim 1, characterized in that the said content is at least 3% by weight.

3. Compositions according to either one of the preceding claims, characterized in that the said content is less than 10% by weight.

4. Compositions according to any one of the preceding claims, characterized in that the anionic surfactant or surfactants are present in an amount of from 5 to 50% by weight, preferably from 5 to 20% by weight, relative to the total weight of the composition.

5. Compositions according to any one of the preceding claims, characterized in that the nonionic surfactant or surfactants are present in an amount of from 5 to 30% by weight, preferably from 5 to 20% by weight, relative to the total weight of the composition.

6. Compositions according to any one of the preceding claims, characterized in that the conditioning agent or agents are present in an amount of from 0.01 to 10% by weight relative to the total weight of the composition.

7. Compositions according to any one of the preceding claims, characterized in that they are aqueous or aqueous-alcoholic compositions.

8. Compositions according to any one of the preceding claims, characterized in that they have a pH of between 2 and 8.

9. Compositions according to Claim 8, characterized in that the said pH is between 3 and 7.

10. Compositions according to any one of the preceding claims, characterized in that the alpha-hydroxy acid or its derivatives is present in free acid form and/or in the form of one of its combined salts and/or in the form of the corresponding lactide.

11. Compositions according to any one of the preceding claims, characterized in that the alpha-hydroxy acid is chosen from citric acid, tartaric acid and lactic acid.

12. Compositions according to any one of the preceding claims, characterized in that the conditioning agent is chosen from cationic polymers, amphoteric polymers, silicones and silicone derivatives.

13. Use of a composition as defined in any one of the preceding claims for the cleaning and/or care of the hair and/or the scalp.

14. Use according to Claim 13, characterized in that the said hair is sensitized hair.

## Patentansprüche

1. Tensidhaltige Zusammensetzungen zur Haarbehandlung, die in einem kosmetisch akzeptablen Medium mindestens einen anionischen grenzflächenaktiven Stoff, mindestens einen nichtionischen grenzflächenaktiven Stoff und mindestens ein Konditioniermittel enthalten,
dadurch **gekennzeichnet**, daß
sie außerdem mindestens eine Carbonsäure, die in α-Stellung eine Hydroxygruppe (α-Hydroxycarbonsäure) aufweist, oder mindestens ein Derivat dieser Carbonsäuren in einem Mengenanteil von mindestens 2 Gew.-%, bezogen auf die gesamte Zusammensetzung, enthalten.

2. Tensidhaltige Zusammensetzungen nach Anspruch 1, dadurch gekennzeichnet, daß der Mengenanteil mindestens 3 Gew.-% beträgt.

3. Tensidhaltige Zusammensetzungen nach einem der vorhergehenden Ansprüche, dadurch gekennzeichnet, daß der Mengenanteil weniger als 10 Gew.-% beträgt.

4. Tensidhaltige Zusammensetzungen nach einem der vorhergehenden Ansprüche, dadurch gekennzeichnet, daß der oder die anionischen grenzflächenaktiven Stoffe in einem Mengenanteil von 5 bis 50 Gew.-% und vorzugsweise 5 bis 20 Gew.-%, bezogen auf das Gesamtgewicht der Zusammensetzung, enthalten sind.

5. Tensidhaltige Zusammensetzungen nach einem der vorhergehenden Ansprüche, dadurch gekennzeichnet, daß der oder die nichtionischen grenzflächenaktiven Stoffe in einem Mengenanteil von 5 bis 30 Gew.-% und vorzugsweise 5 bis 20 Gew.-%, bezogen auf das Gesamtgewicht der Zusammensetzung, enthalten sind.

6. Tensidhaltige Zusammensetzungen nach einem der vorhergehenden Ansprüche, dadurch gekennzeichnet, daß das oder die Konditioniermittel in einem Mengenanteil von 0,01 bis 10 Gew.-%, bezogen auf das Gesamtgewicht der Zusammensetzung, enthalten sind.

7. Tensidhaltige Zusammensetzungen nach einem der vorhergehenden Ansprüche, dadurch gekennzeichnet, daß es sich um wäßrige oder wäßrig-alkoholische Zusammensetzungen handelt.

8. Tensidhaltige Zusammensetzungen nach einem der vorhergehenden Ansprüche, dadurch gekennzeichnet, daß sie einen pH-Wert im Bereich von 2 bis 8 aufweisen.

9. Tensidhaltige Zusammensetzungen nach Anspruch 8, dadurch gekennzeichnet, daß der pH-Wert im Bereich von 3 bis 7 liegt.

10. Tensidhaltige Zusammensetzung nach einem der vorhergehenden Ansprüche, dadurch gekennzeichnet, daß die α-Hydroxycarbonsäure oder ihre Derivate in Form der freien Säure und/oder in Form eines ihrer Salze und/oder in Form des entsprechenden Lactids vorliegen.

11. Tensidhaltige Zusammensetzungen nach einem der vorhergehenden Ansprüche, dadurch gekennzeichnet, daß die α-Hydroxycarbonsäure unter Citronensäure, Weinsäure und Milchsäure ausgewählt ist.

12. Tensidhaltige Zusammensetzungen nach einem der vorhergehenden Ansprüche, dadurch gekennzeichnet, daß das Konditioniermittel unter kationischen Polymeren, amphoteren Polymeren, Siliconen und Siliconderivaten ausgewählt ist.

13. Verwendung einer tensidhaltigen Zusammensetzung nach einem der vorhergehenden Ansprüche für die Reinigung und/oder die Pflege der Haare und/oder der Kopfhaut.

14. Verwendung nach Anspruch 13, dadurch gekennzeichnet, daß die Haare sensibilisierte Haare sind.
